# EUROPÄISCHE PATENTSCHRIFT

(11) **EP 2 611 455 B1**
(45) Veröffentlichungstag und Bekanntmachung des Hinweises auf die Patenterteilung: **11.10.2017**
(21) Anmeldenummer: 11748637.3
(22) Anmeldetag: 19.08.2011
(51) Int. Cl.: A61K 38/17, A61K 38/44, A61P 31/04, A61K 31/095

(54) **STOFFKOMBINATION ZUR BEHANDLUNG VON ENTZÜNDLICHEN ODER INFEKTIÖSEN ERKRANKUNGEN**
MATERIAL COMBINATION FOR TREATING INFLAMMATORY OR INFECTIOUS DISEASES
COMBINAISON DE SUBSTANCES POUR TRAITER DES MALADIES INFLAMMATOIRES OU INFECTIEUSES

(30) Priorität: 02.09.2010 DE 102010040153
(43) Veröffentlichungstag der Anmeldung: 10.07.2013
(73) Patentinhaber: Robert Bosch Gesellschaft MbH Für Medizinische Forschung, 70376 Stuttgart (DE)
(72) Erfinder: WEHKAMP, Jan, 70372 Stuttgart (DE); SCHROEDER, Bjoern, 70376 Stuttgart (DE); STANGE, Eduard, 70469 Stuttgart (DE); NUDING, Sabine, 73061 Ebersbach (DE)
(74) Vertreter: Bee, Joachim
(86) Internationale Anmeldenummer: PCT/EP2011/064318
(87) Internationale Veröffentlichungsnummer: WO 2012/028479

(56) Entgegenhaltungen:
- WO-A2-2006/076742
- WO-A2-2006/103037
- WO-A2-2007/081486
- US-A1- 2004 126 369
- US-B2- 7 312 189
- BJOERN O. SCHROEDER ET AL: "Reduction of disulphide bonds unmasks potent antimicrobial activity of human [beta]-defensin 1", NATURE, Bd. 469, Nr. 7330, 20. Januar 2011 (2011-01-20), Seiten 419-423, XP55016876, ISSN: 0028-0836, DOI: 10.1038/nature09674
- NUDING S ET AL: "A flow cytometric assay to monitor antimicrobial activity of defensins and cationic tissue extracts", JOURNAL OF MICROBIOLOGICAL METHODS, ELSEVIER, AMSTERDAM, NL, Bd. 65, Nr. 2, 1. Mai 2006 (2006-05-01), Seiten 335-345, XP027926797, ISSN: 0167-7012 [gefunden am 2006-05-01]
- NUDING S ET AL: "Antibacterial activity of human defensins on anaerobic intestinal bacterial species: a major role of HBD-3", MICROBES AND INFECTION, ELSEVIER, PARIS, FR, Bd. 11, Nr. 3, 1. März 2009 (2009-03-01), Seiten 384-393, XP026032277, ISSN: 1286-4579, DOI: 10.1016/J.MICINF.2009.01.001 [gefunden am 2009-01-14] in der Anmeldung erwähnt
- M M WELLING ET AL: "Antibacterial activity of human neutrophil defensins in experimental infections in mice is accompanied by increased leukocyte accumulation.", JOURNAL OF CLINICAL INVESTIGATION, Bd. 102, Nr. 8, 15. Oktober 1998 (1998-10-15), Seiten 1583-1590, XP55016871, ISSN: 0021-9738, DOI: 10.1172/JCI3664

## Beschreibung

Die vorliegende Erfindung betrifft eine Stoffkombination zur Behandlung von entzündlichen und infektiösen Erkrankungen des Menschen, die durch obligat oder fakultativ anaerobe Mikroorganismen verursacht werden, sowie ein dieses Stoffkombination enthaltendes Arzneimittel.

Anaerobe Mirkoorganismen, insbesondere Bakterien und Hefepilze, also einzellige Mikroorganismen, die ohne Sauerstoff leben bzw. Sauerstoff-tolerant sind, können verschiedene Krankheitsbilder hervorrufen, wie beispielsweise Wundinfektionen und Abszesse, Sepsis, Infektionen, und zwar insbesondere im Bauchraum, im Harn- und Genitaltrakt oder im Kieferbereich. So finden sich oftmals bereits im Bereich der Mundhöhle, insbesondere in entzündeten Zahnhalteapparaten, derartige pathogene Spezies, sowie im Bereich des Magens in den Magenschleimhautfalten, und im Zwölffingerdarm, die dann lokale, aber auch unter Umständen systemische akute wie auch chronische Entzündungen hervorrufen können. Auch im eher dünn besiedelten Dünndarm können eine Reihe von fakultativen Anaerobiern pathologische Veränderungen der hochempfindlichen Schleimhaut des Dünndarms hervorrufen; im Mast- und Enddarm, den Hauptorten der Bakterienflora, überwiegen zwar aerobe Bakterien, jedoch sind auch hier anaerobe Vertreter ebenfalls in der Lage, schwere Entzündungsreaktionen der Dickdarmschleimhaut zu verursachen. Auch Candida ssp. werden bei vielen Individuen im Stuhl gefunden und sind potentiell pathogen.

Gegenwärtig werden zur Behandlung solcher Erkrankungen insbesondere Antibiotika eingesetzt, die überwiegend die Zellwände der Bakterien angreifen und zerstören. Ein großes Problem, das bei der Therapie mit Antibiotika solcher entzündlicher Erkrankungen auftritt, ist die in jüngerer Zeit immer weiter voranschreitende Resistenzbildung gegenüber den eingesetzten Antibiotika. Diese ermöglicht es den pathogenen Bakterien/Mikroorganismen, die Wirkung von antibiotisch aktiven Substanzen abzuschwächen oder ganz zu neutralisieren. Wenn sich dann ein Mikroorganismus als resistent gegen die gängigen Antibiotika erweist, können Erkrankungen lebensbedrohlich werden. Die Ursache, warum in den vergangenen Jahren die Zahl von multiresistenten Bakterienstämmen stark angestiegen ist, liegt u.a. darin, dass die Bakterien aufgrund ihres raschen Wachstums und ihrer kurzen Kultivierungsperiode die Möglichkeit haben, ständig neue Strategien für die Neutralisierung der Antibiotika zu entwickeln. Daher werden gegenwärtig neben Antibiotika bspw. auch natürliche, insbesondere pflanzliche, sowie synthetische Öle und Emulsionen eingesetzt.

Nach wie vor besteht aber ein großes Bedürfnis an wirksamen Behandlungsmöglichkeiten solcher entzündlichen, durch obligat oder fakultativ anaerobe Mirkoorganismen, insbesondere Bakterien oder Hefepilze (Candida), hervorgerufenen Erkrankungen, sowie insbesondere auch an Alternativen zu Antibiotika, die zu deren Behandlung eingesetzt werden.

Aufgabe der vorliegenden Erfindung ist es daher, einen neuen bzw. alternativen Therapieansatz bereitzustellen, mit welchem durch (an-)aerobe Mikroorganismen, inkl. Candida, hervorgerufenen Erkrankungen effektiv behandelt werden können.

Erfindungsgemäß wird diese Aufgabe durch eine Stoffkombination gelöst, die a) zumindest ein durch eine reduzierende Substanz aktivierbares antimikrobielles Peptid, wobei das Peptid eni humanes beta-Defensin 1 ist oder ein Fragment davon ist, welches Fragment noch die antimikrobielle Aktivität und Funktion des vollständigen humanen beta-Defensin 1 besitzt, und b) zumindest ein Reduktionsmittel enthält, zur Behandlung oder Vorbeugung von entzündlichen und infektiösen Erkrankungen, die durch obligat oder fakultativ anaerobe Mikroorganismen, insbesondere Bakterien und/oder Candida (Hefepilze), verursacht werden.

Die Aufgabe wird ferner gelöst durch ein Arzneimittel, das die erfindungsgemäße Stoffkombination enthält.

Mit der erfindungsgemäßen Stoffkombination können die aufgeführten Erkrankungen und Infektionen erfolgreich bekämpft bzw. diesen vorgebeugt werden. Die Erfinder der vorliegenden Anmeldung konnten in diesem Zusammenhang zeigen, dass die beanspruchten antimikrobiellen Defensine erst in Kombination mit einem Reduktionsmittel aktiviert bzw. antimikrobiell aktiv werden. Durch die gemeinsame Verabreichung dieser beiden Inhaltsstoffe wird somit *in situ -* oder aber durch eine Aktivierung der antimikrobiellen Defensine durch Zugabe des Reduktionsmittels außerhalb des Körpers und vor Behandlung/Vorbeugung - ein zur Behandlung/Vorbeugung von Erkrankungen geeignetes Stoffkombination bereitgestellt, welche durch obligat oder fakultativ anaerobe Bakterien/Hefepilze verursacht werden.

Vorliegend wird unter einem "antimikrobiellen Peptid das durch eine reduzierende Substanz aktivierbar ist", jedes antimikrobielle Peptid, insbesondere ein Defensin, verstanden, das in seiner oxidierten Form nicht antimikrobiell wirksam ist, und welchem erst durch Wirkung einer reduzierenden Substanz die antimikrobielle Wirkung verliehen wird. Dabei wird, wie im betreffenden Fachgebiet üblich unter einem "Peptid" eine organische Verbindung verstanden, die aus einer kettenartigen Verknüpfung mehrerer Aminosäuren zu einer definierten Sequenz hervorgeht. Es versteht sich, dass das Peptid dabei natürlich, d.h. isoliert und gereinigt, oder rekombinant oder synthetisch bereitgestellt werden kann, wobei dem Fachmann übliche Verfahren und Methoden zur Bereitstellung von Peptiden bekannt sind.

Unter einer "reduzierenden Substanz" bzw. einem "Reduktionsmittel" - diese Begriffe werden synonym verwendet - wird vorliegend entsprechend jede Substanz verstanden, die durch Bereitstellung von Elektronen Disulfidbrücken in Peptiden lösen können und die dadurch selber oxidiert werden. So wird bspw. vorliegend unter der Oxidoreduktase Thioredoxin eine reduzierende Substanz/ein Reduktionmittel verstanden, da dieses in seiner reduzierten Form zwei SH-Gruppen aufweist, die in der Redoxreaktion mit Peptiden zu einer Disulfidbrücke verknüpft werden. Erfindungsgemäß und gemäß dem allgemeinen Verständnis eines Fachmanns auf dem betreffenden Gebiet, kann die reduzierende Substanz ein chemisches Reduktionsmittel, bspw. DTT oder DTE sein, oder auch ein auch natürlich vorkommende oder rekombinant hergestellte Enzyme, wie bspw. Oxidoreduktasen.

Vorliegend wird, wie im betreffenden Gebiet allgemein definiert, unter "Defensinen" eine Familie potenter antimikrobieller Peptide verstanden, die durch drei Disulfidbrücken gekennzeichnet sind, und wichtige Schlüsselmoleküle der angeborenen Immunität darstellen, die den Menschen vor Mikroorganismen schützen, und die darüber hinaus die Zusammensetzung der Mikrobiota von Schleimhäuten formen. Von Defensinen ist bekannt, dass sie gegen Bakterien, Pilze und Viren wirken, und zwar indem sie an deren Membranen binden und dadurch deren Permeabilität erhöhen. Basierend auf ihrer Sequenzhomologie und den jeweils vorhandenen Cystein-Resten werden die humanen Defensine in alpha-Defensine and beta-Defensine klassifiziert.

Aufgrund des Vorkommens der Defensine in Schleimhäuten des Menschen ist eine kombinatorische Verabreichung der Defensine oder Fragmenten davon mit einer reduzierenden Substanz, bspw. DTT oder einer Oxidoreduktase, ein geeignetes therapeutisches Mittel für sämtliche entzündlichen und infektiösen Erkrankungen aller Organe, die durch eine Schleimhaut ausgekleidet sind. Dies sind alle inneren Organe, die mit der äußeren Umwelt in Verbindung stehen, also Magen, Darm, Harn- und Atemwege, sowie die weiblichen Reproduktionsorgane. Aufgrund der Tatsache, dass Defensine in den Schleimhäuten des Menschen natürlich vorkommen, ebenso wie viele Oxidoreduktasen, wie von den Erfindern gezeigt, bestehen durch die Verabreichung der Stoffkombination zur Behandlung/Vorbeugung von entzündlichen/infektiösen Erkrankungen von mit einer Schleimhaut ausgekleideten Organen keine Gefahr von Nebenwirkungen. Aufgrund des ubiquitären Vorliegens der Defensine in den Schleimhäuten und deren nachgewiesene Aktivierung durch reduzierende Substanzen ist ferner der Wirkungsmechanismus bei entzündlichen/infektiösen Erkrankungen bzw. bei der Vorbeugung in den genannten Organen derselbe.

Vorliegend versteht sich, dass in der erfindungsgemäßen Stoffkombination nicht nur jeweils ein Defensin, sondern auch eine Kombination von zwei oder mehreren Defensinen vorliegen kann.

Ferner versteht sich, dass nicht nur die vollständigen humanen beta-Defensine 1, sondern auch Fragmente davon, die noch die antimikrobielle Aktivität und Funktion der vollständigen Defensine besitzen, und durch reduzierende Substanzen aktiviert werden können, geeignete antimikrobielle Peptide darstellen, die im Rahmen der vorliegenden Erfindung in der erfindungsgemäßen Stoffkombination eingesetzt werden können. Dem Fachmann sind hinreichende Mittel und Verfahren bekannt, um geeignete Fragmente der Defensine zu finden, insbesondere durch C- oder N-terminale Verkürzung der Defensine, sowie deren antimikrobielle Wirksamkeit zu testen. So betrifft die vorliegende Erfindung bspw. und insbesondere auch ein Fragment, das von zwischen 5 und 15, vorzugsweise 7 C-terminalen Aminosäuren des Defensins HBD1 aufweist (GKAKCCK; SEQ ID Nr. 1) bzw. aus diesen besteht.

Entsprechend ist in einer bevorzugten Ausführungsform das zumindest eine Defensin ausgewählt aus der Gruppe umfassend natürliche, isolierte und gereinigte humane beta-Defensine 1, rekombinante humane beta-Defensine 1, synthetische humane beta-Defensine 1, oder Fragmenten davon. Zur Isolierung natürlich vorkommender Defensine, sowie zur Herstellung rekombinanter oder synthetisch hergestellter Defensine sind dem Fachmann im Stand der Technik hinreichende Möglichkeiten bekannt, die sämtlich in seinem Können und Wissen liegen und übliche Verfahrenstechniken im betreffenden Gebiet darstellen.

Insbesondere ist bevorzugt, wenn das antimikrobielle Defensin ausgewählt ist aus humanem beta-Defensin 1 (hBD-1).. Für hBD-1, das eines der am häufigsten vorkommenden Peptide seiner Klasse ist, wurde im Zusammenhang mit der vorliegenden Erfindung gezeigt, dass dieses in seiner oxidierten Form gegen Laktobazillen, Bifidobakterien und *Candida* keine oder nur eine geringe antibiotische Aktivität aufwies und im Gegensatz dazu eine herausragende antimikrobielle Wirkung in seiner reduzierten Form besitzt, die durch gleichzeitige Gabe von Thioredoxin oder mit chemischen Reduktionssubstanzen herbeigeführt wurde.

Gemäß einer weiteren bevorzugten Ausführungsform ist das Reduktionsmittel eine Oxidoreduktase, insbesondere Thioredoxin, oder eine chemische Reduktionssubstanz, wie bspw. DTT oder DTE. Geeignete reduzierende Substanzen sind ferner Thioredoxin-Reduktase, Protein-Disulfid-Isomerase, Glutaredoxin und Glutathion. Thioredoxin ist als ebenfalls ubiquitär vorkommende Disulfid-Oxidoreduktase, die über die Reduzierung von Disulfidbrücken von Enzymen deren Aktivität und damit eine Reihe von Zellprozessen kontrolliert und reguliert.

Die Verabreichung von Thioredoxin bei entzündlichen Darmerkrankungen wurde zwar bereits in der US 7,312,189 beschrieben, allerdings wird hier lediglich der Einsatz von Thioredoxin als Monopräparat vorgeschlagen. Die erhöhte Wirksamkeit einer Kombination von Thioredoxin bzw. Oxidoreduktasen allgemein und Defensinen wird in dieser Druckschrift weder offenbart noch nahegelegt.

Es wird auch die Verwendung von Thioredoxin als Aktivator von antimikrobiellen Defensinen zur Behandlung oder Vorbeugung von entzündlichen oder infektiösen Erkrankungen beschrieben.

Wie bereits weiter oben ausgeführt, können mit dem Einsatz von Thioredoxin, bspw. in Kombinationspräparaten, Defensine gezielt antimikrobiell aktiviert werden. Die Aktivierung kann dabei durch gemeinsame Verabreichung von Thioredoxin und Defensin erfolgen - wobei die Aktivierung dann *in vivo*/*in situ* erfolgt, oder aber durch vorherige - *ex vivo -* Kombination, und anschließender Verabreichung des dadurch aktivierten Defensins, wobei dieses vor Verabreichung ggf. noch stabilisiert werden kann.

Die zu behandelnden infektiösen bzw. entzündlichen Erkrankungen können dabei durch ein Mikroorganismus ausgelöst sein, der bspw. ausgewählt ist aus *Bifidobacterium* sp., *Lactobacillus* sp., *Escherichia coli, Pseudomonas sp.,* und *Staphylococcus sp., Streptococcus sp. oder Candida sp..*

*Bifidobacterium* sp., *Lactobacillus* sp., *Escherichia coli,* gehören zu der normalen Flora des Darms; *Pseudomonas aeruginosa* und *Staphylococcus aureus* sind bei gefährlichen Lungenerkrankungen wie Cystische Fibrose und Mukoviszidose beteiligt, und *Streptococcus mutans* ist der bedeutendste kariogene Erreger im Mundraum. *Candida albicans* kann im Stuhl vorhanden sein und ist fakultativ pathogen (z.B. bei Immundefekten).

Es versteht sich, dass die obige Aufführung bestimmter Bakterienstämme nicht abschließend sein soll bzw. ist, ebenso wenig wie die Nennung bestimmter Vertreter der einzelnen Spezies. Für den Fachmann ist es basierend auf der Offenbarung dieser Erfindung naheliegend und klar, dass die vorliegend beanspruchte Stoffkombination nicht nur gegen diese anaeroben Mikroorganismen erfolgreich eingesetzt werden kann, sondern auch gegen andere, an entzündlichen und infektiösen Erkrankungen beteiligten anaeroben Bakterien/Mikroorganismen inkl. *Candida*-Spezies, ohne dass deren Aufzählung hier notwendig wäre.

Entsprechend kann das erfindungsgemäße Stoffkombination zur Behandlung bzw. Vorbeugung von Erkrankungen eingesetzt werden, die ausgewählt sind aus chronisch entzündlichen Darmerkrankungen, Karies, Lungenerkrankungen, Erkrankungen des Urogenitaltraktes, Erkrankungen der Bauchspeicheldrüse, Erkrankungen des weiblichen Reproduktionstraktes. All diese Organe zeichnen sich, wie bereits weiter oben erwähnt, durch deren Auskleidung durch Schleimhäute aus, so dass deren Erkrankungen auf die gleiche Weise durch die erfindungsgemäße Kombination behandelt werden können.

Dabei ist insbesondere bevorzugt, wenn die erfindungsgemäße Zusammensetzung zur Behandlung von Morbus Crohn oder Colitis ulcerosa eingesetzt wird. Beide Erkrankungen stellen chronisch entzündliche Darmerkrankungen dar, die durch die Kombination aus aktivierbarem Defensin und Oxidoreduktase behandelt werden können.

Erfindungsgemäß kann die Stoffkombination in einem/als Arzneimittel vorliegen, und zwar in einer pharmazeutisch wirksamen Menge, zusammen mit einem pharmazeutisch akzeptierbaren Träger, Verdünnungsmittel oder Hilfsstoff, und/oder ggf. mit weiteren pharmazeutisch wirksamen Stoffen.

Pharmazeutisch akzeptable Träger mit ggf. weiteren Zusätzen sind im Stand der Technik allgemein bekannt und werden bspw. in der Abhandlung von Kibbe A., Handbook of Pharmaceutical Excipients, Third Edition, American Pharmaceutical Association and Pharmaceutical Press 2000, beschrieben. Zusätze umfassen erfindungsgemäß jedwede Verbindung oder Zusammensetzung, die für eine therapeutische Verwendung der Zusammensetzung vorteilhaft sind, worunter Salze, Bindemittel, Lösungsmittel, Dispersionsmittel, und weitere im Zusammenhang mit der Formulierung von Arzneimitteln üblicherweise verwendete Stoffe fallen.

Das erfindungsgemäße Stoffkombination/Arzneimittel kann dabei in eine für die jeweilige Therapie geeignete Verabreichung integriert werden. Beispiele für eine Verabreichung umfassen parenterale, z.B. intravenöse, intradermale, subkutane, transdermale, transmukosale Verabreichungen. Dabei liegt es im Können und Wissen des Fachmanns, welche Mengen und welche Verabreichungsformen und -wege die jeweils geeigneten sind. Diese können im Hinblick auf die Patienten, die spezifische Erkrankung und unter Berücksichtigung anderer Faktoren variieren.

Entsprechend wird auch ein Verfahren zur Behandlung bzw. zur Vorbeugung von entzündlichen und infektiösen Erkrankungen beschrieben, die durch obligat oder fakultativ anaerobe Bakterien verursacht werden, wobei dem Patienten eine therapeutisch effektive Menge des erfindungsgemäßen Stoffkombination, wie weiter oben beschrieben, verabreicht wird.

Es versteht sich, dass die vorstehend genannten und die nachstehend noch weiter zu spezifizierenden Merkmale nicht nur in der jeweils angegebenen Kombination, sondern auch in anderen Variationen oder in Alleinstellung möglich sind, ohne den Rahmen der vorliegenden Erfindung zu verlassen.

Die Erfindung wird in dem nachstehenden Beispiel und in den Figuren näher erläutert. Es zeigen:

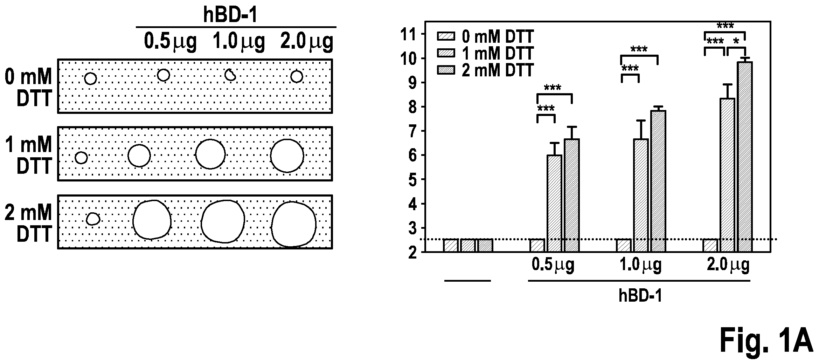

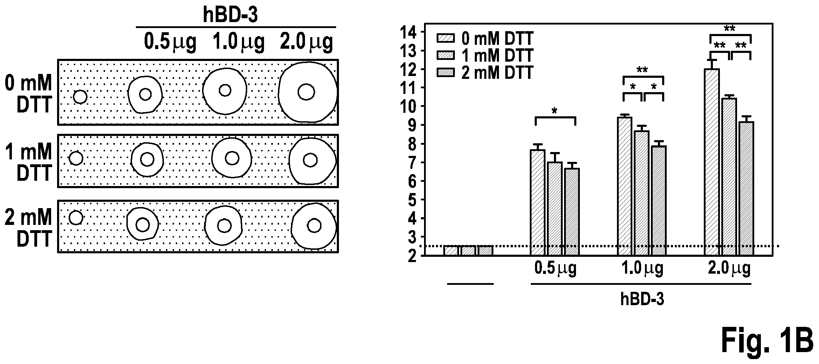

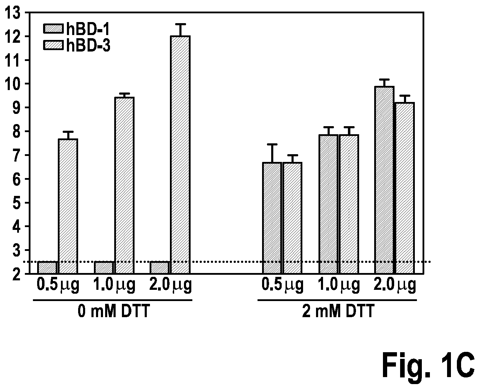

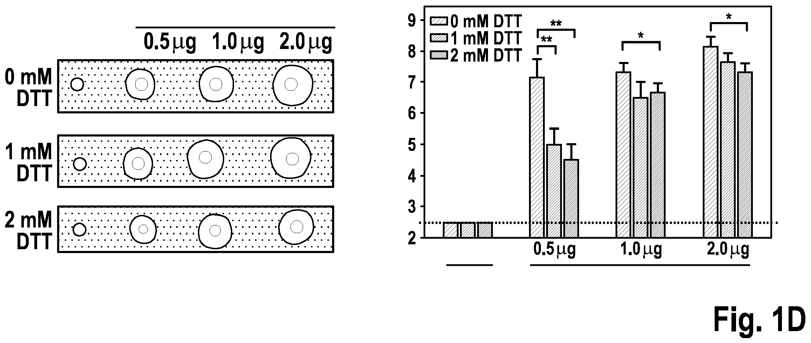

- Fig. 1: die Ergebnisse der Untersuchung der antimikrobiellen Aktivität von hBD-1 gegen *Bif. adolescentis* unter reduzierenden Bedingungen; *Bif. adolescentis* wurde ohne und mit DTT (1 mM oder 2 mM DTT) unter anaeroben Bedingungen mit verschiedenen Konzentrationen an hBD-1 (**A**), hBD-3 (**B**) oder Lysozym (**D**) inkubiert. Die Durchmesser der Inhibierungsbereiche wurden nach Inkubation für 48 Stunden bei 37°C ausgemessen und statistisch evaluiert (GraphPad Prism 4.03 und Student's t-test mit (*) p < 0.05, (**) p < 0.01, (***) p < 0.001. Die Daten sind Mittelwerte, die Balken geben die Standardabweichung wieder; in (**C**) ist der Vergleich der Inhibierungsbereiche zwischen hBD-1 und hBD-3 in Wachstumsmedium ohne oder mit 2 mM DTT gezeigt; die gepunktete Linien bei 2,5 mm stellen die Basislinie für den Durchmesser der Wells dar; hBD-1 (**A**) zeigt antimikrobielle Aktivität erst durch Reduktion, hBD-3 (**B**) und Lysozym (**D**) zeigen unter reduzierenden Bedingungen eine leicht verminderte antimikrobielle Aktivität gegen *Bif. adolescentis*

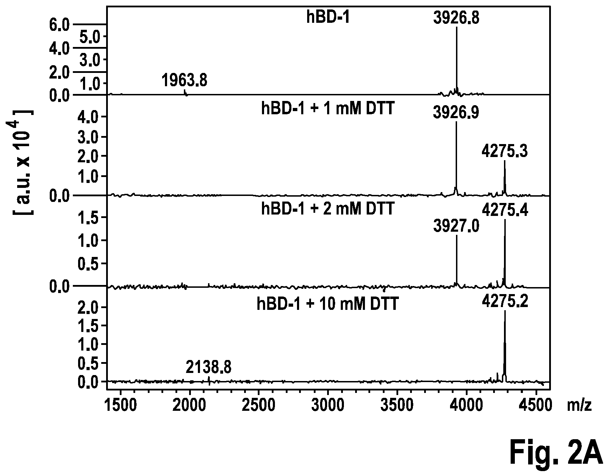

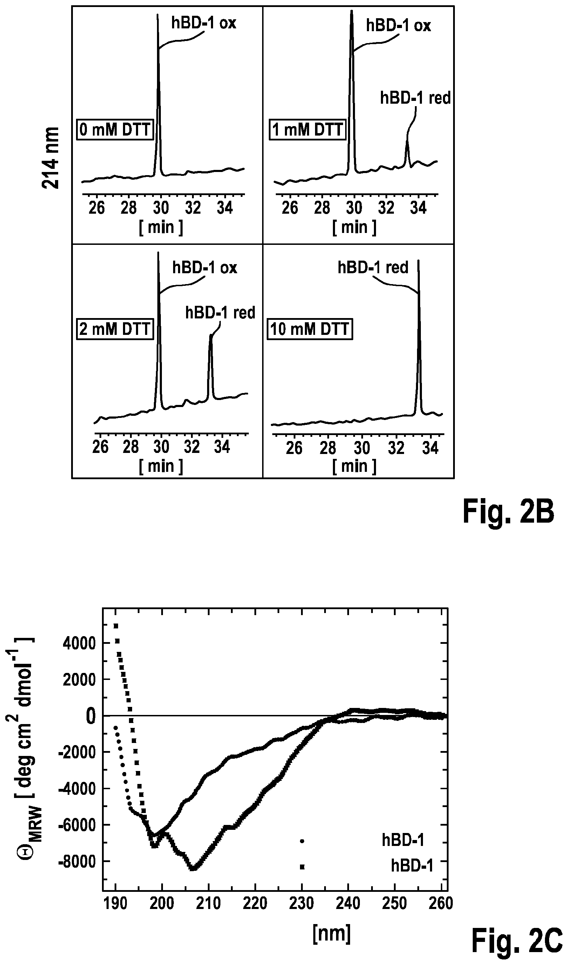

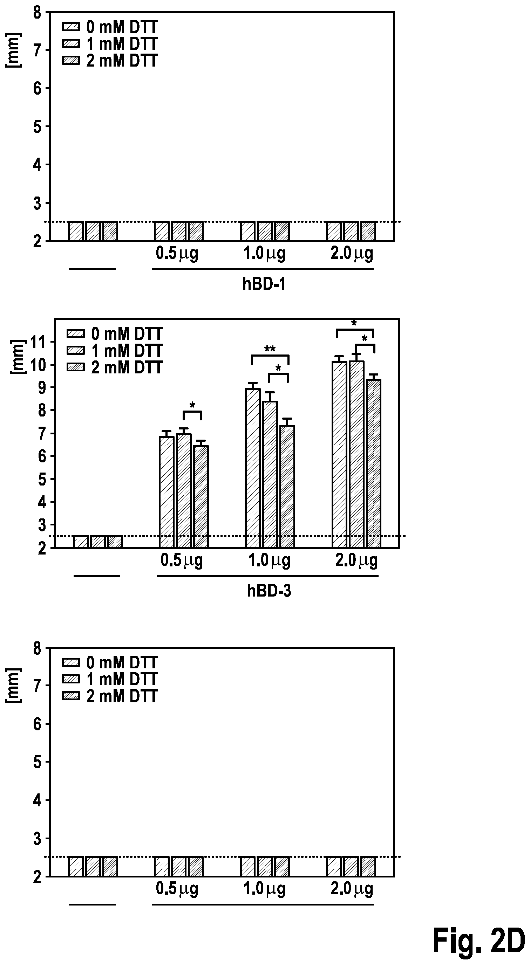

- Fig. 2: die Ergebnisse zu Untersuchungen des oxidierten und des reduzierten hBD-1, wonach reduziertes hBD-1 hydrophober war und eine unterschiedliche Sekundärstruktur hatte im Vergleich zu oxidiertem hBD-1; (**A**) 1 µg hBD-1 wurde mit unterschiedlichen Konzentrationen an DTT inkubiert, mit lodacteamid alkyliert und mittels MALDI-MS analysiert; (**B**) 1 µg hBD-1 wurde mit unetscheidlichen Konzentrationen an DTT inkubiert und mit HPLC analysiert; hBD-1 ox = vollständig oxidiertes hBD-1, hBD-1 red = vollständig reduziertes hBD-1; (**C**) Zirkulardichroismus-Spektroskopie von oxidiertem und reduziertem hBD-1 (0.1 mg/ml) bei 25°C in 10 mM Natriumphosphat-Puffer, pH 7.4; (**D**) hBD-1 zeigte unter reduzierenden Bedingungen keine antimikrobielle Aktivität gegen *Bacteroides vulgatus*; *B. vulgatus* wurde ohne oder mit DTT (1 mM oder 2 mM) unter anaeroben Bedingungen mit 0,5 µg, 1,0 µg oder 2,0 µg hBD-1 (oberes Diagramm), hBD-3 (Mitte) oder Lysozym (unteres Diagramm) inkubiert. Nach Inkubation für 48 bei 37°C wurden die Durchmesser wie für Fig. 1 beschrieben ausgemessen.

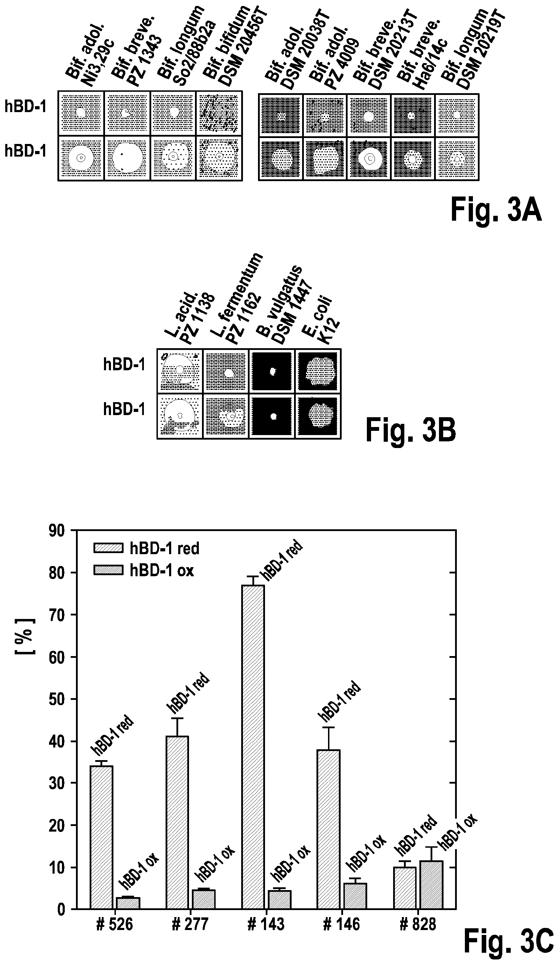

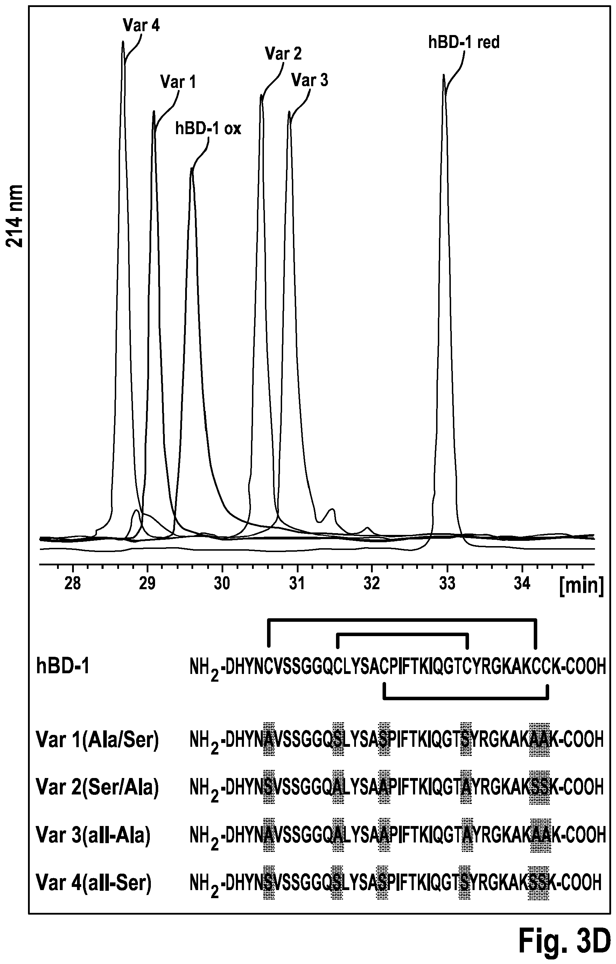

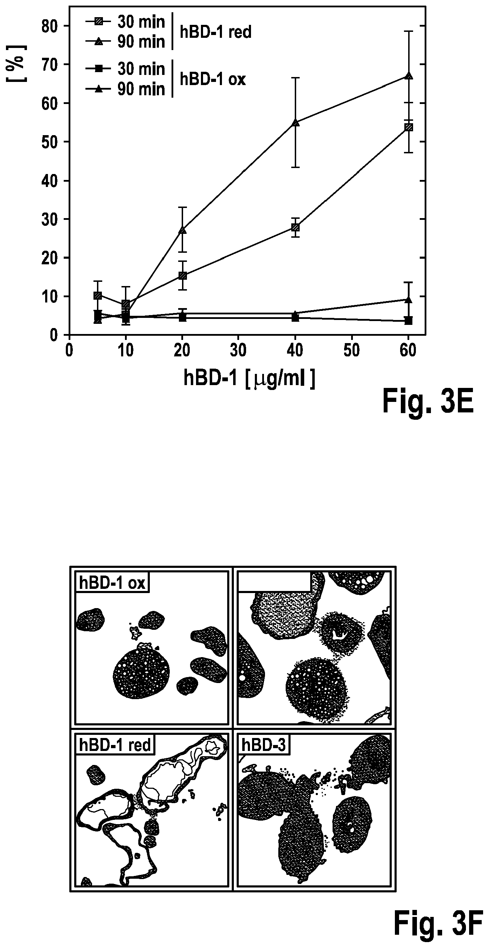

- Fig. 3: Reduziertes hBD-1 zeigt bakterizide Aktivität gegen verschiedene Bifidobakterien Laktobazillen und Candida durch Depolarisation der Zellmembran, was bei Bifidobakterien in zellulärer Desintegration endet. (**A**) 1.5 µg oxidiertes und reduziertes hBD-1 wurden mit *Bifidobacterium spp., Lactobacillus spp., Bacteroides vulgatus* (**B**) und *Escherichia coli* inkubiert. Für Laktobazillen betrug die Defensin-Menge 3 µg. Die Bildung von Inhibitionsbereichen wurde nach 48 Stunden untersucht, repräsentative Radialdiffusionsassays aus 3 Experimenten sind gezeigt.(**C**) Reduziertes hBD-1 (40 µg/ml) wirkt gegen 4 von 5 klinischen Candida-Isolaten wohingegen das oxidierte hBD-1 keine Wirkung zeigt. Gezeigt ist der Anteil depolarisierter Hefen nach 90-minütiger Inkubation mit reduziertem oder oxidiertem hBD-1, gemessen mit einem durchflusszytometrischen Assay. hBD-1 red = reduziertes hBD-1, hBD-1 ox = oxidiertes hBD-1. Die Mittelwerte + Standardabweichung zweier Experimente, durchgeführt jeweils als Duplikate, sind gezeigt, (**D**) Cystein-Aminosäuren im hBD-1 Peptid wurden gegen Alanin oder Serin ausgetauscht und die Hydrophobizität der hBD-1-Varianten wurde per RP-HPLC analysiert. (**E**) Durchflusszytometrischer Assay zur Detektion bakterieller Membran-Depolarisation von *Bifidobakterium adolescentis* nach Inkubation mit reduziertem oder oxidiertem hBD-1. Quadrate zeigen eine Inkubationszeit von 30 Minuten, Kreise eine Zeit von 90 Minuten. Schwarze Symbole zeigen oxidiertes hBD-1, rote Symbole reduziertes hBD-1. Ergebnisse sind als Mittelwerte ± Standardabweichung von zwei Experimenten, jeweils durchgeführt als Duplikate, gezeigt. (**F**), Transmission-Elektronenmikroskopische Aufnahme von *Bifidobacterium adolescentis* nach Inkubation für 2 Stunden mit 200 µg/ml oxididiertem hBD-1 (hBD-1 ox, oben links), ohne Defensin (Kontrolle, oben rechts), mit 200 µg/ml reduziertem hBD-1 (hBD-1 red, unten links) oder mit 200 µg/ml hBD-3 (unten rechts). Fixierte Bakterien wurden mittels Transmissions-Mikroskopie analysiert, Vergrößerungsbalken = 200 nm.

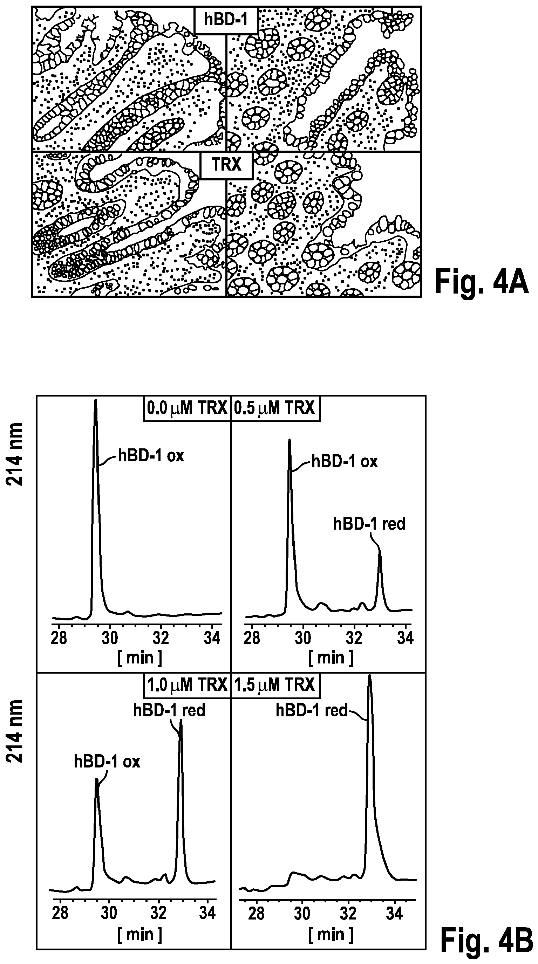

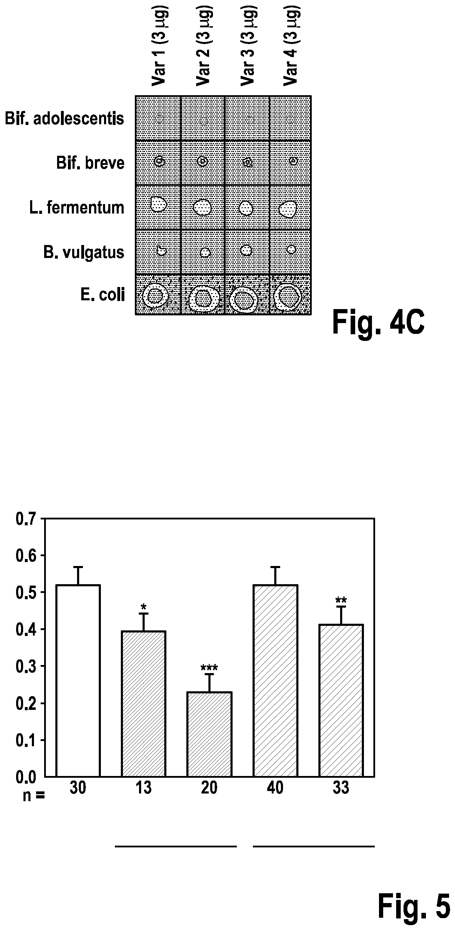

- Fig. 4: Thioredoxin (T-RX) ko-lokalisiert mit hBD-1 und ist in der Lage die Reduktion von hBD-1 zu katalysieren. (**A**) Immunohistochemie-Färbung von hBD-1 und Thioredoxin von humanen Kolon-Schnitten. Vergrößerungsbalken = 100 µm. (**B**) 10 µM oxidiertes hBD-1 wurde ohne oder mit 0.5,1.0 und 1.5 µM humanem Thioredoxin und 100 nM Ratten-Thioredoxinreduktase für 30 Minuten bei 37°C in 0.10 M Kaliumphosphat-2 mM EDTA, pH 7.0 Puffer und 0.8 mM NADPH inkubiert. Inkubationsansätze wurden mittels RP-HPLC analysiert und die Umwandlung von oxidiertem in reduziertes hBD-1 wurde beobachtet. hBD-1 ox = oxidiertes hBD-1, hBD-1 red = reduziertes hBD-1. (**D**) Antimikrobielle Aktivität der Ala/Ser-Varianten von hBD-1 (3 µg) gegen *Bifidobacterium adolescentis* Ni3,29c, *Bif. breve* PZ 1343,*Lactobacillus fermentum* PZ 1162, *Bacteroides vulgatus* DSM1447 und *Escherichia coli* K12, gemessen mit dem Radialdiffusionsassay. Bildung von Inhibitionsbereichen wurde nach Inkubation für 48 Stunden untersucht, repräsentative Assays sind gezeigt.
- Fig. 5: Expression von Thioredoxin mRNA in humanen Kolon-Biopsien von gesunden Kontrollpatienten und Patienten mit chronischentzündlichen Darmerkrankunken (nicht-entzündet und entzündet). Thioredoxin mRNA-Expression wurde mittels realtime PCR analysiert, mit gesunden Kontrollen verglichen und auf beta-Aktin normalisiert. Die Daten wurden statistisch ausgewertet mit GraphPad Prism 4.03 und dem Mann-Whitney-Test mit (*) p < 0.05, (**) p < 0.01 und (***) p < 0.001. Daten sind als Mittelwerte ± Standardabweichung gezeigt.

Beispiele: Reduzierung verschiedener Peptide führt zu deren antimikrobiellen Aktivität.

### Material und Methoden:

Die eingesetzten Bakterienstämme wurden von Ardeypharm (Herdecke, Deutschland) sowie von der Deutschen Sammlung für Mikroorganismen und Zellkulturen (DSMZ, Braunschweig, Deutschland) bezogen.

Das Assay (antimikrobielles Radialdiffusions-Assay) hinsichtlich der antimikrobiellen Aktivität wurde wie folgt durchgeführt: Die Bakterien wurden vor dem antimikrobiellen Test anaerob kultiviert. Während der Durchführung des Assays in 10 mM Natriumphosphat mit 0,3 mg/ml of Trypticase Soja Bouillon (TSB) und 1% (w/v) Agarose mit niedriger EEO (Sigma-Aldrich) erfolgte die Inkubation unter anaeroben Bedingungen mit antimikrobiellen Peptiden und 0-2 mM DTT. Das Vorliegen einer reduzierenden Umgebung wurde durch Hinzufügen des Redox-Indikators Resazurin kontrolliert. Nach 48-stündiger Inkubation wurde der Durchmesser der Inhibierungsbereiche gemessen und unter Verwendung von GraphPad Prism 4.03 und Student's t-Test statistisch evaluiert.

Matrix-unterstützte Laser-Desorption/Ionisation Massenspektroskopie (MALDI-MS) wurde wie folgt durchgeführt: hBD-1 wurde mit DTT inkubiert, mit lodacetamid alkyliert und mit alpha-Cyano-4-hydroxy-Zimtsäure ko-kristallisiert. Die MALDI-MS wurde mit einer Bruker Ultraflex TOF/TOF Maschine durchgeführt.

Für die Hochleistungsflüssigkeitschromatographie- (High Performance Liquid Chromatography (HPLC)) Analyse wurde ein System der Agilent 1200 Serie und eine Vydac 218TP-C18 Säule eingesetzt, wobei ein Wasser/Acteonitril Gradient mit Trifluoressigsäure als Ionenpaar-Reagens eingesetzt wurde.

Zirkulardichroismus- (CD) Spektroskopie wurde auf einem Jasco J-715 Spektropolarimeter (Jasco Corporation, Japan) bei 25°C in 10 mM Natriumphosphat, pH 7,4 durchgeführt. Die Spektren wurden bei 0,1 mg/ml im UV-fernen Bereich aufgenommen.

Das Thioredoxin-Reduktaseassay wurde wie folgt beschrieben durchgeführt: Hierzu wurde oxidiertes hBD-1 mit 0,5 -1,5 µM Thioredoxin oder Protein-Disulfid-Isomerase in Gegenwart von 100 nM Thioredoxin-Reduktase and 0,8 mM NADPH in 0,1 M Kalziumphosphatpuffer-2mM EDTA inkubiert. Die Inkubationsmischungen wurden mit RP-HPLC analysiert, wodurch die Umwandlung von dem oxidierten in das reduzierte hBD-1 Peptid beobachtet werden konnte.

Das durchflusszytometrische antimikrobielle Assay, bei dem die Membran-Depolarisierung der Bakterien gemessen wurde, wurde durchgeführt, wie beschrieben in: Nuding, S., et al., "Antibacterial activity of human defensins on anaerobic intestinal bacterial species: a major role of HBD-3." Microbes. Infect. 11 (3), 384-393 (2009).

Für die Transmissionselektronenmikroskopie der Bakterien wurden ca. 2 x 10⁸ CFU (Colony forming units; Kolonie bildende Einheiten) von *Bifidobacterium adolescentis* Ni3,29c, anaerob über zwei Stunden bei 37°C in Abwesenheit oder Gegenwart von 200 µg/ml synthetischem Defensin inkubiert. Die Bakterien wurden zentrifugiert, in vorgewärmter Karnovsky's Lösung fixiert und 24 Stunden bei 4°C gelagert. Nach einer Zentrifugation wurde das Pellet in 3,5% Agarose bei 37°C eingebettet, bei Raumtemperatur koaguliert, in kleine Blöcke geschnitten und wieder in Karnovsky's Lösung fixiert. Die Nach-Fixierung basierte auf 1,0% Osmiumtetroxid mit 1,5% K-Ferrocyanid in 0,1 M Cacodylat-Puffer. Nach Einbettung in Glyzidether wurden die Blöcke mit einem Ultra Mikrotom (Ultracut, Reichert, Wien, Österreich) geschnitten. Ultradünnschnitte (30 nm) wurden auf Kupfergitternetze aufgebracht und mit einem Zeiss LIBRA 120 Transmissionselektronenmikroskop (Carl Zeiss, Oberkochen, Deutschland) mit 120 kV analysiert.

Immunohistochemische Färbungen wurden mit in Paraffin eingebetteten Schnitten von humanen Darm-Biospien durchgeführt. Kaninchen-anti-hBD1 wurde 1:500 verdünnt; Kaninchen anti-TRX wurde 1:1000 verdünnt. Der Sekundär-Antikörper, anti-Kaninchen, war Peroxidase-gekoppelt, die Detektion wurde mit einem DAKO Detektionssystem durchgeführt.

Isolierung der Gesamt-RNA und Real-time PCR: Die Messung der Thioredoxin m RNA in humanen Darm-Biopsien ist beschrieben in: Wehkamp, J., et al., "NOD2 (CARD15) mutations in Crohn's disease are associated with diminished mucosal alpha-defensin expression." Gut 53 (11), 1658-1664 (2004).

### Ergebnisse:

Das klassische Radialdiffusionsassay (wie beschrieben in Lehrer, R.I., et al., "Ultrasensitive assays for endogenous antimicrobial polypeptides." J. Immunol. Methods 137 (2), 167-173 (1991)) wurde modifiziert, um die antimikrobielle Aktivität von synthetischem humanem beta-Defensin 1 (hBD-1) gegen die bakteriellen Stämme der anaeroben normalen Flora, einschließlich des Gram-positiven Stammes *Bifidobacterium adolescentis* and des Gram-negativen Stammes *Bacteroides vulgatus,* unter anaeroben Bedingungen zu analysieren. Geringe Konzentrationen des reduzierenden Agens Dithiothreitol (DTT) wurden zum Assaymedium hinzugefügt, während Medium ohne DTT als Kontrolle diente. Nach 48 Stunden Inkubation wurde die antimikrobielle Aktivität durch Abmessen der Durchmesser der Inhibierungsbereiche bestimmt. Im Medium ohne DTT hatten unterschiedliche Mengen an hBD-1 keinen Einfluss auf das Wachstum von *Bifidobacterium adolescentis* (Fig. 1A).

Im Medium mit 1 mM DTT konnten Konzentrations-abhängige Inhibierungsbereiche nachgewiesen werden. Dieser Effekt war Dosis-abhängig, da ansteigende DTT-Konzentrationen zu einem Anstieg der Inhibierungsbereiche in Größe und Definiertheit führten. Um auszuschließen, dass DTT die bakterielle Fitness beeinflusste, wurden die gleichen Versuche mit beta-Defensin hBD-3 and Lysozym durchgeführt. hBD-3 zeigte eine starke antimikrobielle Aktivität im Medium ohne DTT und eine verminderte Aktivität nach Hinzufügung von DTT (Fig. 1B); ein ähnlicher Effekt wurde für Lysozym beobachtet (Fig. 1D). Dies zeigt, dass die erhöhte antimikrobielle Aktivität von hBD-1 in einer reduzierenden Umgebung ein spezifischer Effekt war, der nicht auf eine beeinträchtigte bakterielle Fitness zurückzuführen war. Bei Konzentrationen von 2 mM DTT war hBD-1 gegenüber *Bif. adolescentis* so effektiv wie hBD-3 (Fig. 1C). Dieses Ergebnis ist überraschend, da hBD-3 eines der stärksten antimikrobiellen Peptide in Sauerstoff-reicher Umgebung ist, wohingegen hBD-1 als eines der schwächsten eingestuft wurde. Bei einem Test mit dem Gram-negativen anaerobe Stamm *Bacteroides vulgatus* (siehe Fig. 2D) zeigte sich unter keiner der Bedingungen ein Wachstums-inhibierender Effekt, wohingegen hBD-3 Konzentrations-abhängige Inhibierungsbereiche zeigte.

Da beta-Defensine durch drei intramolekulare Disulfid-Brücken charakterisiert sind, wurde als nächstes die Beteiligung der Cystein-Brücken bei der antimikrobiellen Wirkung untersucht. Hierzu wurde hBD-1 mit ansteigenden Konzentrationen an DTT inkubiert, und die Proben mittels MALDI analysiert. Für das oxidierte hBD-1 konnte ein Signal beobachtet werden (berechnete monoisotopische Masse: 3926,8 Da, Fig. 2A). Nach Inkubation mit ansteigenden Konzentrationen an DTT und einer Carboxamidmethylierung, um freie Sulfhydryl (SH)-Gruppen zu detektieren, wurde ein zweiter Peak mit einer Massendifferenz von +348 Dalton detektiert, welcher der vollständig reduzierten Form mit sechs alkylierten Cysteinresten entsprach (-CH₂CONH₂, +58 Dalton). Diese Ergebnisse legen einen Alles-oder-Nichts-Mechanismus nahe, bei welchem alle drei Disulfid-Brücken entweder vorliegen oder nicht, ohne dass ein Zwischenstadium mit einer oder zwei Disulfid-Brücken existieren würde. Konsequenterweise war nach Inkubation mit DTT eine Fraktion von hBD-1 vollständig reduziert, während die zweite Fraktion oxidiert blieb.

Als nächstes wurde eine Umkehrphasen-Hochleistungsflüssigkeitschromatographie (Reversed-phase high performance liquid chromatography (RP-HPLC))-Analyse durchgeführt, um die quantitativen Ergebnisse der MALDI-MS zu verifizieren (Fig. 2B). Während oxidiertes, sämtliche Disulfid-Brücken aufweisendes hBD-1 nach 30 Minuten eluierte, resultierte die Inkubation des hBD-1-Peptids mit ansteigenden Konzentrationen an DTT in einen Shift in Richtung eines zweiten Peaks bei 33.5 Minuten, welcher dem vollständig reduzierten hBD-1 Peptid entsprach (was durch Massenspektroskopie bestätigt wurde, Daten nicht gezeigt). In der RP-HPLC zeigte daher das reduzierte hBD-1 einen Anstieg in der Retentionszeit, was auf einen Anstieg in der Hydrophobizität des linearen Peptids in Lösung hinwies.

Im Folgenden wurde die Sekundärstruktur mit Zirkulardichroismus (CD)-Spektroskopie untersucht (Fig. 2C). Das native hBD-1 Peptid zeigte ein charakteristisches Minimum bei 209 nm für alpha-Helices und ein signifikantes Signal bei 218 nm, was dem beta-Faltblatt-Gehalt entsprach, und was auf ein gut gefaltetes Peptid hinwies. Im Gegensatz dazu zeigte das reduzierte Peptid keines dieser Minima, sondern ein Minimum bei ungefähr 195 nm, was auf eine Konformationsänderung in eine Random-Coil-Struktur hinwies. Tatsächlich legt die Berechnung der Sekundärstrukturelemente mit unterschiedlichen Algorithmen für das reduzierte Peptid einen Anstieg in der Random-Coil-Fraktion nahe. Daher wurde die antimikrobielle Aktivität von hBD-1 in reduzierender Umgebung auf dessen reduzierte, offene Form zurückgeführt.

Um auszuschließen, dass die beobachteten Effekte auf Änderungen der bakteriellen Oberfläche zurückzuführen sind, verursacht durch das reduzierende Agens DTT im Medium, wurde die antimikrobielle Aktivität des oxidierten und des reduzierten hBD-1 gegenüber *Bifidobacterium adolescentis, Bif. breve, Bif. longum* and *Bif. bifidum* unter anaeroben Bedingungen in Medium ohne DTT getestet. Die oxidierte Form von hBD-1 zeigte keine antimikrobielle Aktivität gegenüber Stämmen von *Bifidobacterium spp.* (Fig. 3A). Demgegenüber zeigte das reduzierte hBD-1 Peptid eine antimikrobielle Aktivität gegenüber sämtlichen getesteten Stämmen (Fig. 3A), während die Größe der Inhibierungsbereiche zwischen den Stämmen variierte. Darüber hinaus wurde das gleiche Assay mit den Gram-positiven Stämmen *Lactobacillus acidophilus* and *L. fermentum* durchgeführt, sowie mit dem Gram-negativen Stamm *Bacteroides vulgatus* and dem fakultativ anaeroben Stamm *Escherichia coli* K12 (Fig. 3B). Reduziertes hBD-1 führte zu klaren Inhibierungsbereichen bei den *Lactobacilli,* wohingegen oxidiertes hBD-1 keinerlei Effekt zeigte. Bei dem Gram-negativen Stamm *Bacteroides vulgatus* konnte für keines der hBD-1 ein Effekt beobachtet werden, hinsichtlich *E. coli* konnte eine antimikrobielle Aktivität von sowohl der oxidierten als auch der reduzierten Form beobachtet werden. Dies zeigte, dass hBD-1 durch eine reduzierende Umgebung gegenüber Gram-positiven Anaerobiern der humanen normalen Flora spezifisch aktiviert wird. Bei dem Test hinsichtlich des fakultativ pathogenen Pilzes *Candida albicans* konnte für das reduzierte hBD-1, nicht jedoch für das oxidierte hBD-1 eine wirkungsvolle antifungische Aktivität gegenüber vier von fünf Stämmen nachgewiesen werden (Fig. 3C). Dies zeigt, dass hBD-1 durch eine reduzierende Umgebung nicht nur gegenüber Gram-positiven Anaerobiern der humanen normalen Flora aktiviert wird, sondern auch gegenüber einem fakultativ pathogenen Pilz medizinischer Bedeutung.

Anschließend wurden Ala/Ser-Varianten von hBD-1 generiert, bei welchen alle Cysteinreste durch Alanin- oder Serinreste ausgetauscht wurden, um zu untersuchen, ob eine permanent offene Struktur für die erhöhte Aktivität verantwortlich ist (Fig. 3D). Diese Varianten waren jedoch gegenüber *Bifidobacteria* and *Lactobacilli* entweder inaktiv oder nur sehr schwach aktiv, wobei Peptidmengen von bis zu 3 µg eingesetzt wurden (Fig. 4C).

Das einzige Bakterium, das gegenüber den Varianten 1 bis 4 sensitiv war, war *E*. *coli* K12, das auch gegenüber dem oxidierten hBD-1 sensitiv war (Fig. 3B). Für die Untersuchung der Hydrophobizität der generierten Ala/Ser-Varianten wurden RP-HPLC-Analysen durchgeführt (Fig. 3D). Alle vier Varianten wurden - im Vergleich zu reduziertem hBD-1 - bei niedrigeren Acetonitril-Konzentrationen eluiert, was auf eine niedrigere Oberflächen-Hydrophobizität hinwies. Eine verkürzte Variante von hBD-1, die die sieben C-terminalen Aminosäuren GKAKCCK (hBD-1_{Δ30-36}) (SEQ ID Nr. 1) nicht aufwies, konnte das Wachstum von *Bif. adolescentis* weder unter normalen noch unter reduzierenden Bedingungen inhibieren (Daten nicht gezeigt).

Darüber hinaus verschwand die antimikrobielle Aktivität des vollständigen hBD-1 in Gegenwart von 150 mM Natriumchlorid (Daten nicht gezeigt). Daher ist eine lineare Struktur alleine nicht ausreichend für die antimikrobielle Aktivität von reduziertem hBD-1, wichtig sind vielmehr die Hydrophobizität, Ionen-Wechselwirkungen und freie Cysteinreste im C-Terminus.

Um zu untersuchen, ob die beobachtete Wachstumsinhibierung Grampositiver Anaerobier bakteriostatisch oder bakterizid ist, wurden durchflusszytometrische antimikrobielle Assays mit bakterieller Membran-Depolarisation als Auslesesystem durchgeführt. Dadurch konnten die Ergebnisse des Radialdiffusionsassays bestätigt worden, wonach das reduziert hBD-1 Peptid gegenüber *Bif. adolescentis* verglichen mit der oxidierten Form effektiver war (Fig. 3E). Das reduzierte Peptid verursachte eine Membran-Depolarisation von 50% der Bakterien nach lediglich 30 Minuten Inkubation mit 60 µg/µl, während das oxidierte Peptid keine signifikante Depolarisation zu diesem Zeitpunkt oder nach 90 Minuten zeigte. Dies wies auf einen schnellen bakteriziden Effekt des linearen hBD-1 hin.

Im Folgenden wurde eine Transmissionselektronenmikroskopie mit *Bifidobacterium adolescentis* durchgeführt, um die Aktivität des linearen hBD-1 auf einer morphologischen Basis zu analysieren. Nach Inkubation mit oxidiertem hBD-1 (Fig. 3F, oben links) zeigten die bakteriellen Zellen keinerlei morphologische Schäden, verglichen mit der unbehandelten Kontrolle (oben rechts). Im Gegensatz dazu konnte nach Inkubation mit reduziertem hBD-1 (unten links) eine zelluläre Desintegration beobachtet werden, während die Membran-Strukturen morphologisch intakt blieben. Dieser Prozess startete intrazellulär, bevor er sich über das ganze Bakterium ausbreitete, und zu einem vollständigen Verlust des zytoplasmatischen Inhalts führte. Dies wies wiederum darauf hin, dass reduziertes hBD-1 eher bakterizid als bakteriostatisch wirkte, und zwar durch einen Mechanismus, der sich von hBD-3 unterscheidet, welches überwiegend die bakterielle Membran angreift (unten rechts).

Die physiologische, enzymatische Redox-Regulierung wird hauptsächlich durch Thioredoxin (TRX) kontrolliert, einer multifunktionellen Oxidoreduktase. Da TRX von Schleimhäuten exprimiert wird und auch extrazelluläre Funktionen aufweist, wurde die TRX-Expression ebenso wie die hBD-1-Expression in humanem Darm-Gewebe mittels immunhistochemischen Versuchen untersucht. Wie in Fig. 4A gezeigt, konnte hBD-1 in der Darmschleimhaut nachgewiesen werden, einschließlich des Epithels, das die Darmkrypten auskleidet (oben). Ein ähnliches Färbemuster wurde für Thioredoxin erhalten (Fig. 4A, oben), was darauf hinwies, dass sowohl hBD-1 als auch Thioredoxin *in vivo* ko-lokalisieren.

Um zu analysieren, ob hBD-1 ein natürliches Substrat für Thioredoxin ist, wurde hBD-1 mit unterschiedlichen Konzentrationen an TRX in Gegenwart von NADPH und Thioredoxinreduktase inkubiert, was insgesamt das natürliche Thioredoxin-System darstellt. Nach der Inkubation wurden die Proben mittels RP-HPLC analysiert, um die oxidierten und reduzierten hBD-1 Fraktionen zu untersuchen. Wie in Fig. 4b gezeigt, wurde hBD-1 durch Thioredoxin reduziert, wobei der Prozess Konzentrations-abhängig war. Auch eine Erhöhung der TRX-Reduktase steigerte die Menge an reduziertem hBD-1, während ein Weglassen von entweder TRX oder TRX Reduktase eine Reduktion von hBD-1 verhinderte (Daten nicht gezeigt). Dem Thioredoxin-System ist daher die katalysierende Umwandlung von oxidiertem hBD-1 in reduziertes hBD-1 zuzuschreiben. Niedrige Sauerstoff-Level mit weniger als 1% können in Wunden und infektiösem Gewebe auftreten. Diese Stellen sind für eine Invasion von anaeroben Pathogenen sowie von Bakterien, die das umgebende Epithel bewohnen, äußerst angreifbar.

*Bifidobacteria* sind Gram-positive anaerobe Mikroorganismen, die den humanen Darmtrakt als Hauptkomponente der normalen Flora bewohnen. Auch *Lactobacilli* werden im menschlichen Darmtrakt, der Vagina, und im Mund-/Rachenraum gefunden, welche sämtlich Lokalisationen mit einem geringen Sauerstoffgehalt darstellen.

Mit den vorliegenden Daten wurde gezeigt, dass eine Veränderung der Umgebungsbedinungen die antimikrobielle Aktivität von hBD-1 stark beeinflusst.

Mit den vorliegenden Daten wurde ferner gezeigt, dass hBD-1 in einer anaeroben, reduzierenden Umgebung das Schleimhautepithel gegen eine Besiedelung durch die Bakterien der normalen Flora schützt. Durch eine Aktivierung im Lumen, welche von der Milieuumgebung abhängt, kann eine wirkungsvolle antimikrobielle Aktivität getriggert werden

## Patentansprüche

1. Stoffkombination enthaltend a) zumindest ein durch eine reduzierende Substanz aktivierbares antimikrobielles Peptid, wobei das Peptid humanes beta-Defensin 1 ist, oder ein Fragment davon ist, welches Fragment noch die antimikrobielle Aktivität und Funktion des vollständigen humanen beta-Defensins 1 besitzt, und b) zumindest ein Reduktionsmittel, zur Behandlung oder Vorbeugung von entzündlichen und infektiösen Erkrankungen, die durch obligat oder fakultativ anaerobe Mikroorganismen verursacht werden.

2. Stoffkombination nach Anspruch 1, **dadurch gekennzeichnet, dass** das Defensin oder das Fragment davon ausgewählt ist aus der Gruppe umfassend natürliches, isoliertes und gereinigtes humanes beta-Defensin 1, rekombinantes humanes beta-Defensin 1, synthetisches humanes beta-Defensin 1, oder jeweils Fragmente davon, welche noch die antimikrobielle Aktivität und Funktion des vollständigen humane beta-Defensins 1 besitzen.

3. Stoffkombination nach einem der vorherigen Ansprüche, **dadurch gekennzeichnet, dass** das Reduktionsmittel ausgewählt ist aus chemischen Reduktionsmitteln oder enzymatischen Reduktionsmitteln.

4. Stoffkombination nach einem der vorstehenden Ansprüche, **dadurch gekennzeichnet, dass** das Reduktionsmittel eine Oxidoreduktase ist.

5. Stoffkombination nach einem der Ansprüche 1 bis 3, **dadurch gekennzeichnet, dass** das Reduktionsmittel ausgewählt ist aus Thioredoxin, Thioredoxin-Reduktase, DTT, DTE, Protein-Disulfid-Isomerase, Glutaredoxin und Glutathion.

6. Stoffkombination nach einem der Ansprüche 1 bis 5, **dadurch gekennzeichnet, dass** die entzündliche oder infektiöse Erkrankung durch einen Mikroorganismus verursacht wird, der ein Bakterium oder ein Hefepilz ist.

7. Stoffkombination nach einem der Ansprüche 1 bis 5, **dadurch gekennzeichnet, dass** die entzündliche oder infektiöse Erkrankung durch ein Mikroorganismus verursacht wird, der ausgewählt ist aus *Bifidobacterium* sp., *Lactobacillus* sp., *Escherichia coli, Candida sp..*

8. Stoffkombination nach einem der Ansprüche 1 bis 5, **dadurch gekennzeichnet, dass** die entzündliche oder infektiöse Erkrankung, die durch durch obligat oder fakultativ anaerobe Mikroorganismen verursacht wird, ausgewählt ist aus chronisch entzündlichen Darmerkrankungen, Karies, Lungenerkrankungen, Erkrankungen des Urogenitaltraktes, Erkrankungen der Bauchspeicheldrüse, Erkrankungen des weiblichen Reproduktionstraktes.

9. Stoffkombination Anspruch 8, **dadurch gekennzeichnet, dass** die chronisch entzündliche Darmerkrankung ausgewählt ist aus Morbus Crohn und Colitis ulcerosa.

10. Arzneimittel, enthaltend die Stoffkombination nach einem der Ansprüche 1 bis 5 sowie einen pharmazeutisch akzeptierbaren Träger.

## Claims

1. Material combination containing a) at least one antimicrobial peptide that can be activated by a reducing substance, wherein the peptide is human beta-defensin 1 or a fragment thereof, which fragment still possesses the antimicrobial activity and function of the complete human beta-defensin 1, and b) at least one reducing agent, for treating or preventing inflammatory and infectious diseases that are caused by microorganisms that are obligate or facultative anaerobes.

2. Material combination according to Claim 1, **characterized in that** the defensin or the fragment thereof is selected from the group comprising natural, isolated and purified human beta-defensin 1, recombinant human beta-defensin 1, synthetic human-beta defensin 1, or in each case fragments thereof, which fragments still possess the antimicrobial activity and function of the complete human beta-defensin 1.

3. Material combination according to one of the preceding claims, **characterized in that** the reducing agent is selected from chemical reducing agents or enzymatic reducing agents.

4. Material combination according to one of the preceding claims, **characterized in that** the reducing agent is an oxidoreductase.

5. Material combination according to one of Claims 1 to 3, **characterized in that** the reducing agent is selected from thioredoxin, thioredoxin reductase, DTT, DTE, protein disulfide-isomerase, glutaredoxin and glutathione.

6. Material combination according to one of Claims 1 to 5, **characterized in that** the inflammatory or infectious disease is caused by a microorganism, which is a bacterium or a yeast.

7. Material combination according to one of Claims 1 to 5, **characterized in that** the inflammatory or infectious disease is caused by a microorganism that is selected from *Bifidobacterium* sp., *Lactobacillus* sp., *Escherichia coli, Candida* sp.

8. Material combination according to one of Claims 1 to 5, **characterized in that** the inflammatory or infectious disease caused by microorganisms that are oligate or facultative anaerobes is selected from chronic inflammatory bowel diseases, caries, lung diseases, diseases of the urogenital tract, diseases of the pancreas, diseases of the female reproductive tract.

9. Material combination Claim 8, **characterized in that** the chronic inflammatory bowel disease is selected from Crohn's disease and ulcerative colitis.

10. Medicinal product, containing the material combination according to one of Claims 1 to 5 and a pharmaceutically acceptable carrier.

## Revendications

1. Combinaison de matières contenant a) au moins un peptide antimicrobien activable par une substance réductrice, le peptide étant la bêta-défensine 1 humaine ou un fragment de celle-ci, ledit fragment présentant encore l'activité et la fonction antimicrobienne de la bêta-défensine 1 humaine entière, et b) au moins un réducteur, pour le traitement ou la prévention de maladies inflammatoires et infectieuses, qui sont causées par des microorganismes anaérobies stricts ou facultatifs.

2. Combinaison de matières selon la revendication 1, **caractérisée en ce que** la défensine ou le fragment de celle-ci est choisi dans le groupe comprenant la bêta-défensine 1 humaine naturelle, isolée et purifiée, la bêta-défensine 1 humaine recombinante, la bêta-défensine 1 humaine synthétique ou des fragments de chacune, qui présentent encore l'activité et la fonction antimicrobienne de la bêta-défensine 1 humaine entière.

3. Combinaison de matières selon l'une quelconque des revendications précédentes, **caractérisée en ce que** le réducteur est choisi parmi les réducteurs chimiques ou les réducteurs enzymatiques.

4. Combinaison de matières selon l'une quelconque des revendications précédentes, **caractérisée en ce que** le réducteur est une oxydoréductase.

5. Combinaison de matières selon l'une quelconque des revendications 1 à 3, **caractérisée en ce que** le réducteur est choisi parmi la thiorédoxine, la thiorédoxine réductase, le DTT, le DTE, la protéine disulfure isomérase, la glutarédoxine et la glutathione.

6. Combinaison de matières selon l'une quelconque des revendications 1 à 5, **caractérisée en ce que** la maladie inflammatoire ou infectieuse est causée par un microorganisme qui est une bactérie ou une levure.

7. Combinaison de matières selon l'une quelconque des revendications 1 à 5, **caractérisée en ce que** la maladie inflammatoire ou infectieuse est causée, par un microorganisme qui est choisi parmi *Bifidobacterium* sp., *Lactobacillus* sp., *Escherichia coli, Candida* sp..

8. Combinaison de matières selon l'une quelconque des revendications 1 à 5, **caractérisée en ce que** la maladie inflammatoire ou infectieuse qui est causée par des microorganismes anaérobies stricts ou facultatifs est choisie parmi les maladies intestinales inflammatoires chroniques, les caries, les maladies pulmonaires, les maladies des voies urogénitales, les maladies du pancréas, les maladies des voies reproductrices de la femme.

9. Combinaison de matières la revendication 8, **caractérisée en ce que** la maladie intestinale inflammatoire chronique est choisie parmi la maladie de Crohn et la colite ulcéreuse.

10. Médicament, contenant la combinaison de matières selon l'une quelconque des revendications 1 à 5 et un véhicule pharmaceutiquement acceptable.


### SEQUENZ PROTPKOLL

<110> Robert Bosch Gesellschaft mbH für medizinische Forschung
<120> Stoffkombination zur Behandlung von entzündlichen oder infektiösen Erkrankungen
<130> 333073
<160> 6
<170> PatentIn version 3.3
<210> 1
   <211> 7
   <212> PRT
   <213> Künstliche Sequenz
<220>
   <223> Synthetisches Peptid: C-Terminus hBD-1
<400> 1
   Gly Lys Ala Lys Cys Cys Lys
   1               5
<210> 2
   <211> 36
   <212> PRT
   <213> Homo sapiens
<400> 2
   Asp His Tyr Asn Cys Val Ser Ser Gly Gly Gln Cys Leu Tyr Ser Ala
   1               5                   10                  15
   Cys Pro Ile Phe Thr Lys Ile Gln Gly Thr Cys Tyr Arg Gly Lys Ala
               20                  25                  30
   Lys Cys Cys Lys
           35
<210> 3
   <211> 36
   <212> PRT
   <213> Künstliche Sequenz
<220>
   <223> Synthetisches Peptid: hBD-1 Variante 1
<400> 3
   Asp His Tyr Asn Ala Val Ser Ser Gly Gly Gln Ser Leu Tyr Ser Ala
   1               5                   10                  15
   Ser Pro Ile Phe Thr Lys Ile Gln Gly Thr Ser Tyr Arg Gly Lys Ala
               20                  25                  30
   Lys Ala Ala Lys
                                 35
<210> 4
   <211> 36
   <212> PRT
   <213> Künstliche Sequenz
<220>
   <223> Synthetisches Peptid: hBD-1 Variante 2
<400> 4
   Asp His Tyr Asn Ser Val Ser Ser Gly Gly Gln Ala Leu Tyr Ser Ala
   1               5                   10                  15
   Ala Pro Ile Phe Thr Lys Ile Gln Gly Thr Ala Tyr Arg Gly Lys Ala
               20                  25                  30
   Lys Ser Ser Lys
           35
<210> 5
   <211> 36
   <212> PRT
   <213> Künstliche Sequenz
<220>
   <223> Synthetisches Peptid: hBD-1 Variante 3
<400> 5
   Asp His Tyr Asn Ala Val Ser Ser Gly Gly Gln Ala Leu Tyr Ser Ala
   1               5                   10                  15
   Ala Pro Ile Phe Thr Lys Ile Gln Gly Thr Ala Tyr Arg Gly Lys Ala
               20                  25                  30
   Lys Ala Ala Lys
           35
<210> 6
   <211> 36
   <212> PRT
   <213> Künstliche Sequenz
<220>
   <223> Synthetisches Peptid: hBD-1 Variante 4
<400> 6
   Asp His Tyr Asn Ser Val Ser Ser Gly Gly Gln Ser Leu Tyr Ser Ala
   1               5                   10                  15
   Ser Pro Ile Phe Thr Lys Ile Gln Gly Thr Ser Tyr Arg Gly Lys Ala
               20                  25                  30
   Lys Ser Ser Lys
               35